Europäisches Patentamt

European Patent Office

Office européen des brevets

(19)

(11) Veröffentlichungsnummer: **0 451 791 A2**

(12) # EUROPÄISCHE PATENTANMELDUNG

(21) Anmeldenummer: 91105615.8

(22) Anmeldetag: 09.04.91

(51) Int. Cl.⁵: **A61K 9/127**, A61K 37/02

(30) Priorität: **12.04.90 DE 4011864**

(43) Veröffentlichungstag der Anmeldung:
**16.10.91 Patentblatt 91/42**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Kibat, Paul-Gerhard, Dr.**
**Gartenstrasse 17**
**W-6238 Hofheim am Taunus(DE)**
Erfinder: **Sandow, Jürgen Kurt, Dr.**
**Am Heideplacken 22**
**W-6240 Königstein/Taunus(DE)**

(54) Langwirksame Liposomenpräparate von Peptidarzneistoffen und Verfahren zu ihrer Herstellung.

(57) Es werden Lipsomenpräparate für Peptide mit verlängerter Peptidfreisetzung beschrieben. Die Peptide weisen ein Molekulargewicht zwischen 500 und 10000 auf, die Phospholipidkomponente der Liposomenmembran besitzt eine Phasenübergangstemperatur von mindestens 20° C und enthält überwiegend gesättigte Fettsäuren. Die Wirksamkeit nach s.c. oder i.m. Injektion hält mehr als 14 Tage an. Ferner werden Verfahren zur Herstellung dieser Liposomenpräparate beschrieben.

EP 0 451 791 A2

Die Erfindung bezieht sich auf langwirksame Liposomenpräparate von Peptidarzneistoffen zur parenteralen Applikation. Die erfindungsgemäßen Präparationen werden subkutan (s.c.) oder intramuskulär (i.m.) appliziert und haben eine Wirkdauer von mehr als 14 Tagen. Ferner betrifft die Erfindung auch Verfahren zur Herstellung dieser Präparate.

Liposomen sind submikroskopische Teilchen von hohlkugelartiger Gestalt. Sie besitzen eine Doppelmembran, die aus amphiphilen Molekülen, meist Phospholipiden, aufgebaut ist und einen wäßrigen Innenraum umgibt. Sie bestehen aus körperähnlichem Material, können als Träger unterschiedlichster Substanzen fungieren und spezifischen Anforderungen gezielt angepaßt werden. Dabei werden hydrophile Arzneistoffe zum überwiegenden Teil im wäßrigen Innenvolumen eingeschlossen, während lipophile Substanzen zum größten Teil in der Membran gebunden sind.

Liposomen werden als Trägersysteme für eine Vielzahl von Arzneistoffen vorgeschlagen, wie z.B. für Zytostatika, Antiinfektiva und Immunmodulatoren (z.B. Yatvin, M.B. und Lelkes, P.I., Med. Phys. 9, (1982)). Hauptsächlich werden liposomale Arzneimittel parenteral angewandt, wobei oft die intravenöse Gabe angestrebt wird. Zielsetzung ist meist die Nutzung eines Depoteffektes, die Reduzierung von Nebenwirkungen oder die Erhöhung der Wirksamkeit. Nach i.v. Injektion werden Liposomen, wie alle kolloidalen Systeme, von den Zellen des retikuloendothelialen Systems (RES) aufgenommen, mit einer Halbwertszeit von maximal 2 Tagen eliminiert und bevorzugt in Leber und Milz angereichert. (Senior, J.H., CRC, Critical Reviews in Therapeutic Drug Carrier Systems 3, 123 (1987)). Nach s.c. oder i.m. Injektion werden im Vergleich zu i.v. Injektionen längere Wirkspiegel erhalten. Für Liposomenpräparate ist die Wirkdauer abhängig von der Substanzfreigabe aus den Vesikeln und Von ihrem Abtransport von der Injektionsstelle sowie vom Abbau der Vesikel. Substanzfreigabe und Abbau werden vor allem von der Zusammensetzung der Liposomenmembran bestimmt, während der Abtransport von der Teilchengröße abhängig ist, d.h. mit abnehmender Teilchengröße zunimmt (Arrowsmith et al., Int. J. Pharm. 20, 347-362 (1984)). Ein zusätzlicher Faktor ist die Lipidkonzentration im Präparat (Jackson, A.J., Res. Comm. Chem. Pathol. Pharmacol. 27, 293 (1980)).

Die in den vorstehend angegebenen Publikationen beschriebenen Untersuchungen zur i.m. oder s.c. Applikation von liposomalen Arzneistoffträgern zeigten in keinem Falle eine Arzneistofffreigabe oder einen Verbleib des Präparates am Applikationsort über mehr als 14 Tage. Vielmehr zeigten auch die pharmakokinetischen Untersuchungen zu diesen Liposomenpräparationen verschiedener Vesikel-Zusammensetzung und verschiedener Arzneistoffe, daß im Zeitraum von 14 Tagen entweder die Wirkstofffreigabe beendet war oder daß innerhalb dieser Zeit die Liposomen abgebaut waren.

Liposomenpräparate zur s.c. oder i.m. Injektion für Peptide sind bereits beschrieben worden. Eine Liposomenformulierung zur Langzeitfreigabe von Insulin ist z.B. in GB-B-2.050.287 dargestellt. Die internationale Patentanmeldung mit der Veröffentlichungs-Nr. WO 87/04592 beschreibt ein Liposomenfreigabesystem für membraninpermeable Moleküle - im Beispiel Calcitonin-, das aus einer Mischung von kleinen wirkstoffhaltigen SUV (unilamellare Liposomen, Teilchengröße etwa 30-100 nm) mit großen MLV (multilamellare Vesikel, Teilchengröße etwa 200-10000 nm) besteht. Fukunaga et al. (Endocrinology 115, 757(1984)) beschreibt einen verlängerten hypocalcämischen Effekt von Calcitonin nach liposomaler Verkapselung des Proteins. Entsprechend den Beispielen in diesen Publikationen konnte in keinen Fall eine Wirksamkeit über mehr als 14 Tage festgestellt werden.

Für Peptide, wie z.B. für LHRH-Analoga, werden langwirksame Formulierungen auf der Basis von bioabbaubaren Polymeren beschrieben, (vgl. z.B. EP-B 0 052 510 und EP-B 0 145 240 für Mikrokapseln, EP-B 0 058 481 für andere kontrollierte Freigabesysteme). EP-A 0 299 402 beschreibt langwirksame Formulierungen von LHRH-Analogen mit antagonistischer Aktivität.

Während in den 4 vorstehend genannten Schriften Liposomenformulierungen nicht erwähnt sind, beschreibt GB-B 2 050 287 eine LHRH-haltige Liposomenzusammensetzung, die jedoch im Gegensatz zu den Zusammensetzungen der vorliegenden Erfindung Freigabemodulatoren enthält und eine Eliminationshalbwertszeit von ca. 4 Tagen nach s.c.-Injektion hat.

Liposomen als Trägersysteme für LHRH wurden auch von Schäfer et al. (Pharmazie 42, 674 (1987) und Pharmazie 42, 689 (1987)) beschrieben. Sie stellten MLV aus Mischungen von Eilecithin und Phosphatidsäure her und untersuchten die Pharmakokinetik nach i.m.-Applikation an Kaninchen bzw. Schweinen. Die Halbwertszeit der Elimination von der Injektionsstelle betrug maximal 20 Stunden. LHRH-Blutspiegel waren nach dieser Zeit nicht mehr meßbar.

Überraschenderweise zeigt sich mit den nachstehend charakterisierten, besonderen Liposomenformulierungen, daß sie z. T. noch nach 35 Tagen am Injektionsort nachweisbar sind und zu signifikanten Blutspiegeln bzw. pharmakologischen Effekten führen.

Die Erfindung betrifft daher Liposomenpräparate für Peptide mit verlängerter Peptidfreisetzung, dadurch gekennzeichnet, daß die Peptide ein Molekulargewicht zwischen etwa 500 und 10000 aufweisen, die

EP 0 451 791 A2

Phospholipidkomponente der Liposomenmembran eine Phasenübergangstemperatur von mindestens 20˚ C besitzt und überwiegend gesättigte Fettsäuren enthält und daß die Wirksamkeit nach s.c. oder i.m. Injektion mehr als 14 Tage anhält.

Die erfindungsgemäßen Liposomenszubereitungen gewährleisten aufgrund ihrer besonderen Zusammensetzung eine Wirksamkeit über einen Zeitraum von mehr als 14 Tage. Das bedeutet, daß die Präparate aufgrund ihrer speziellen Zusammensetzung sowohl über mehr als 14 Tage am Applikationsort verbleiben ohne abgebaut zu werden als auch über diesen Zeitraum die eingeschlossenen Peptidwirkstoffe in für eine gewünschte Wirksamkeit genügender Menge freigeben.

Die Wirksamkeit hält vorzugsweise mindestens 20 Tage, insbesondere 30 Tage und länger an.

Die mittlere volumenäquivalente Teilchengröße der Vesikel (Liposomen) liegt bevorzugt zwischen 600 nm und 10000 nm, insbesondere über 800 Nanometer, um die Geschwindigkeit des Abtransportes von der Injektionsstelle zu minimieren. Die Phospholipidkomponente der Liposomenmembran hat vorzugsweise eine Phasenübergangstemperatur von über 30˚ C, insbesondere von mindestens 37˚ C. Sie enthält überwiegend gesättigte Fettsäuren mit einer Kettenlänge von mindestens 14 Kohlenstoffatomen.

Geeignete Phospholipide sind z.B. Dimyristoyl-PC (DMPC), Distearoyl-PC (DSPC), Dipalmitoyl-PC (PC = Phosphatidyl-Cholin) oder hydrierte bzw. teilhydrierte Lecithine aus natürlichen Quellen. Zur Stabilisierung der Membran sind z.B. lipophile Zusätze von Steroiden, wie Cholesterol, geeignet.

Die in Liposomen verkapselte Peptide (auch in Form ihrer physiologisch verträglichen Salze) sind natürlichen, synthetischen oder halbsynthetischen Ursprungs u. besitzen spezifische Wirkungen im Organismus. In den vorstehenden und folgenden Ausführungen werden daher im Sinne der Erfindung unter Peptiden sowohl freie Verbindungen als auch die physiologisch verträglichen Salze der wie vorstehend charakterisierten Peptide verstanden. Sie haben ein Molekulargewicht von etwa 500 bis 10000. Geeignete Peptide sind z.B. LHRH-Analoga, Bradykinin-Antagonisten, Insulin, Vasopressin, Oxytocin, Calcitonin, Heparin, Hirudin und ihre synthetischen oder semisynthetischen Analoga. Vorzugsweise werden LHRH-Analoga verkapselt, wie z.B. Buserelin, HOE 013 (Ac-D-Nal-p-Cl-D-Phe-D-Trp-Ser Tyr-D-Ser ($\alpha$-L-Rha)-Leu-Arg-Pro-Azagly-$NH_2$, vgl. EP-A 0 263 521, entspr. US-Patentanmeldung No. 390477). Es eignen sich aber auch z.B. Hirudine wie HBW 023 (R-DNA-Hirudin gem. EP-A-0 324 712, entspr. US-Patentanmeldung No. 295 422), HOE 427 (= Ebiratide, [4-Methionindioxid, 8-D-Lysin, 9-Phenylamin]-$\alpha$-MSH-(4-9)-(8-amino-octyl)amid-triacetat (vgl. EP-A-0 179 332 entspr. US-Patente No. 4,623,715 und No. 4,696,913) und HOE 140 (= H-D-Arg-Arg-Pro-Hyp-Gly-Thi-Ser-D-Tic-Oic-Arg-OH•$6CH_3COOH$ (vgl. EP-A-0 370 453 entspr. US-Patentanmeldung No. 374 162).

Es ist bekannt, daß die als Wirkstoffe geeigneten Peptide nach der Applikation im lebenden Organismus nur sehr kurzzeitig wirksam sind (Banga et al., Int. J. Pharm. 45, 15-50 (1988)). Sie werden durch Enzyme oder auch chemische Reaktionen inaktiviert bzw sehr schnell eliminiert. Durch die Verkapselung dieser Peptide zu den erfindungsgemäßen Liposomenpräparate ist es möglich, die Substanzen vor der schnellen metabolischen Inaktivierung im Organismus zu schützen und eine langanhaltende kontinuierliche Freigabe des unveränderten Wirkstoffes über längere Zeiträume sicher zu stellen.

Die Liposomen sind entweder von unilamellaren oder multilamellaren Typ. Die Peptide können sich sowohl im wäßrigen Innenraum als Lösung als auch in der Liposomenmembran befinden. Die Wirkstofffreigabe wird insbesondere über die Membran gesteuert, d.h. ihre Beschaffenheit und eventuell der Wirkstoffanteil in der Membran beeinflussen die Dauer der Wirkstofffreigabe. Durch Verkapselung von Peptiden in große, z.B. multilamellare, Liposomen wird z.B. die Wirkdauer durch die Bindung des Wirkstoffs an das Trägersystem erhöht z.B. auf 20 Tage und länger. Bei den erfindungsgemäßen Liposomenpräparaten sind z.B. auch nach 30 Tagen an der Injektionsstelle noch Liposomen aufzufinden. Auch eine Wirksamkeit ist nach diesem Zeitraum nachweisbar.

Durch Zusätze von negativ oder positiv geladenen Ladungsträgern wie z.B. Dipalmitoyl-Phosphatidyl-Glycerol oder Stearylamin im Membranteil, Antioxidantien oder anderen Hilfsstoffen mit stabilisierenden oder freigabebeeinflussenden Eigenschaften, kann die Wirkstofffreigabe zusätzlich gesteuert werden.

Die Liposomen können prinzipiell nach allen Methoden, wie sie z.B. aus der Literatur (Lichtenberg, D., Methods of Biochemical Analysis 33, 337 (1988)) bekannt sind, hergestellt werden. Insbesondere sind Herstellungstechnologien, die größere Liposomen liefern, geeignet.

Die Verfahren zur Herstellung der erfindungsgemäßen Liposomenpräparate sind dadurch gekennzeichnet, daß man

a)

$\alpha$) die Phospholipidkomponente, und gegebenenfalls lipophile Zusätze, in einem geeigneten organischen Lösungsmittel löst, das Lösungsmittel entfernt und die erhaltene Lipidmatrix nach Zusatz einer wäßrigen Lösung des Peptids zur Liposomenbildung ablöst, wobei die Ablösung oberhalb der Phasenübergangstemperatur der Phospholipidkomponente erfolgt, oder

3

β) die Phospholipidkomponente, und gegebenenfalls lipophile Zusätze, sowie das Peptid in einem geeigneten organischen Lösungsmittel löst, das Lösungsmittel entfernt und die erhaltene Lipidmatrix mit einem wäßrigen Medium ablöst, wobei die Ablösung oberhalb der Phasenübergangstemperatur der Phospholipidkomponente erfolgt, oder

γ) die Phospholipidkomponente, und gegebenenfalls lipophile Zusätze, in einem flüchtigen organischen Lösungsmittel löst und eine wäßrige, mit der organischen Phase nicht mischbare Peptidlösung zusetzt, das erhaltene Zwei-Phasensystem durch Homogenisierung oberhalb der Phasenübergangstemperatur der Phospholipidkomponente in eine stabile Emulsion überführt und das organische Lösungsmittel unter Bildung von Liposomen entfernt

und die nach den Methoden a bis γ erhaltenen Liposomendispersionen, gegebenenfalls nach Homogenisierung und Äquilibrierung auf den gewünschten Peptidgehalt einstellt, abfüllt und gegebenenfalls gefriertrocknet, oder

b) ein Lyophilisat von Peptid-freien Liposomen nach Methoden α, β oder γ herstellt und eine wäßrige Peptidlösung in einem geeigneten Gefäß abfüllt, wobei Lyophilisat und Peptidlösung vor Applikation vereinigt werden.

Die nach den erfindungsgemäßen Verfahren erhaltenen Lyophilisate werden nach üblichen Methoden wie z.B. Zusatz von Aqua. ad Injektabila in für i.m. oder s.c. geeignete Applikationsformen überführt.

Das bei dem erfindungsgemäßen Verfahren verwendete wäßrige Medium besteht aus Wasser oder einem Gemisch aus Wasser und einem organischen Lösungsmittel wie z.B. Methanol oder Ethanol. Es kann außerdem noch Zusätze wie Kochsalz oder Puffer, z.B. Phosphatpuffer enthalten. Auch die wäßrigen Peptidlösungen können solche Zusätze aufweisen.

Die Verfahren werden zweckmäßigerweise wie folgt durchgeführt.

Methode a)

α) Phospholipide und gegebenenfalls lipophile Zusätze (z.B. Cholesterol) werden in einem organischen Lösungsmittel, wie z.B. Ethanol, Methanol, Dichlormethan, Chloroform, tert. Butanol, gelöst. Das Lösungsmittel wird mit Methoden entfernt, die keine bedenklichen Lösungsmittel-Rückstände zulassen und eine Lipidmatrix mit möglichst großer Oberfläche ergeben. Besonders geeignet sind hierzu eine Evakuation mit rotierenden Verdampfern und die Lyophilisation oder Kombinationen der Methoden.

Zur Liposomenbildung wird die Lipidmatrix nach dem Zusatz einer wäßrigen, erforderlichenfalls gepufferten Lösung des Peptidarzneistoffs abgelöst. Dieser Prozeß muß bei Temperaturen des Ansatzes durchgeführt werden, die oberhalb der Phasenübergangstempeatur der Phospholipidkomponente und selbstverständlich unterhalb einer kritischen Zersetzungstemperatur des Peptids liegen. Er wird durch Agitation des Gefäßes und durch den Einsatz beschleunigender Hilfsmittel (z.B. Glasperlen oder Abstreifer) unterstützt. Die Liposomendispersion kann anschließend noch einem Homogenisierungsschritt, z.B. mit Ultraturrax, Hochdruckhomogenisatoren und vergleichbaren Verfahren, unterworfen werden. Die gebildeten Liposomen werden bei erhöhter Temperatur solange äquilibriert, bis sie einen stabilen Zustand und optimale Quellung erreicht haben. Die Homogenität der Dispersion wird durch die Abtrennung von Grobanteilen verbessert, indem sie z.B. durch Membran- oder Glasfilter von 1-20 μm Porendurchmesser filtriert wird.

Bei nicht quantitativer Verkapselung des Arzneistoffes ergibt sich in vielen Fällen die Notwendigkeit zur Abtrennung des unverkapselten Anteils. Besondere Vorteile bei der Trennung von gebundenem und freiem Arzneistoff bietet eine Cross-Flow Filtration, die bei geeigneter Wahl der Membranen auch eine Abtrennung des Feinanteiles an Liposomen (kleiner ca. 400 nm) erlaubt. Daneben können Zentrifugationsverfahren, chromatographische Verfahren (Gel-, Ionenaustausch oder Absorptionschromatographie) oder eine Abtrennung des freien Peptides mit adsorbtiven oder digestiven Methoden angewendet werden.

Die fertige Liposomendispersion wird mit geeigneten Methoden bezüglich der Arzneistoffkonzentration untersucht und auf den erforderlichen Gehalt verdünnt. Sie wird in Ampullen oder Injektionsfläschchen abgefüllt und bei geeigneten Bedingungen gelagert.

Alle Verfahrensschritte werden bei der Herstellung pharmazeutischer Präparationen unter aseptischen Bedingungen durchgeführt.

β) Die Herstellung erfolgt analog Methode α, jedoch wird das Peptid zusammen mit den lipophilen Bestandteilen im organischen Lösungsmittel gelöst. Dieses Verfahren ist für Peptide mit lipophilem Charakter besonders geeignet; als Lösungsmittel bieten sich Ethanol, Methanol und tert. Butanol an.

γ) Phospholipide und lipophile Zusätze (z.B. Cholesterol) werden in einem leicht flüchtigen, organischen Lösungsmittel, wie z.B. Diethylether, Diisopropylether oder deren Mischung mit Dichlormethan bzw. Chloroform gelöst. Zu dieser Lösung wird eine wäßrige, mit der organischen Phase nicht mischbare Peptidlösung gegeben. Das Zwei-Phasensystem wird mit geeigneten Homogenisierverfahren (Ultraturrax, Ultraschall, Hochdruckhomogenisator) bei Temperaturen oberhalb der Phasenübergangstemperatur der Phospholipidkomponente und unterhalb einer kritischen Zersetzungstemperatur des Peptids in eine stabile Emulsion überführt. Danach wird das organische Lösungsmittel bei der erforderlichen Temperatur unter Vakuum entfernt. Über einen metastabilen, meist gelartigen Zwischenzustand bilden sich die Liposomen, die durch weiteren Lösungsmittelentzug weitgehend von Verunreinigungen befreit werden. Die Liposomen werden wie unter Methode α beschrieben weiterverarbeitet, aufgereinigt und abgefüllt.

Liposomen, die nach Methode α-γ hergestellt werden und in der wäßrigen Lösung Zusätze von kryoprotektiven Substanzen enthalten, oder denen nach der Herstellung Kryoprotektiva zugesetzt wurden, können gefriergetrocknet werden. Die Wahl der Zusatzstoffe und Gefriertrocknungsverfahren sind so abgestimmt, daß die Liposomen vor der Applikation leicht rekonstituierbar sind und einen großen Anteil des Peptidarzneistoffs in gebundener Form enthalten. Geeignete Kryoprotektiva sind zum Beispiel Mannitol, Xylitol, Sorbitol, Trehalose, Dextrane, Polyvinylpyrrolidon, Albumin, Hydroxyethylstärke und modifizierte Gelatinetypen.

Methode b)

Wirkstofffreie Liposomen werden entsprechend Methode a) α, β oder γ hergestellt und wie vorstehend beschrieben lyophilisiert. Zur Herstellung der Liposomendispersion wird dem Lyophilisat sterile, wäßrige Peptidlösung zugesetzt. Diese Liposomendispersion kann dann appliziert werden.

Wirkstofffreie Liposomen, die nach Methode a) α β oder γ gewonnen werden, werden gegebenenfalls durch geeignete Homogenisierungsverfahren in Dispersionen von kleinen Vesikeln überführt. Ein besonders geeignetes Verfahren ist die Hochdruckhomogenisation, z.B. mit einem Mikrofluidizer, daneben kann aber auch eine Behandlung mit Ultraschall oder Ultraturrax erfolgen. Die hierbei entstehenden kleinen Liposomen können anschließend einer Entkeimungsfiltration unterworfen werden, bevor sie wie oben beschrieben lyophilisiert werden. Diese Liposomen werden vor Applikation mit der Peptidlösung vereinigt. Die so erhaltene Dispersion weist überwiegend große Visikel auf.

Die erfindungsgemäßen Liposomenpräparate weisen eine lang andauernde, kontinuierliche Wirkstofffreigabe auf. Sie zeichnen sich ferner durch ihre hohe Stabilität bei der Lagerung aus. So ist, wie in Beispiel 9 beschrieben, nach 12-monatiger Lagerung noch mehr als 99 % des Peptids liposomengebunden, und die Teilchengröße ist unverändert.

Beispiel 1

200 mg LHRH-Antagonist (HOE 013), 1348 mg hydriertes Eilecithin (Phasenübergangstemperatur etwa 53 °C) und 652 mg Cholesterol werden in 50 ml Methanol bei 50 °C gelöst. Die Lösung wird durch 0,2 μm Membranfilter steril filtriert und unter aseptischen Bedingungen zu Liposomen verarbeitet. Dazu wird das Lösungsmittel am Rotationsverdampfer bei 55 °C entfernt, bis sich eine dünne Lipidmatrix (Film) bildet. Der Lipidfilm wird unter Stickstoffbegasung mit 20 ml steriler Kochsalzlösung versetzt, innerhalb 2 Stunden bei 55 °C von der Gefäßwand abgelöst und über Nacht bei 50 °C geschüttelt. Die erhaltene Liposomendisper-sion wird durch 5 μm Membranfilter filtriert und mit Kochsalzlösung auf 100 ml aufgefüllt. Die erhaltene Dispersion wird in Polycarbonat-Zentrifugengläser überführt und bei 20.000 x g über 5 Minuten bei 5 °C zentrifugiert. Der Überstand mit gelöstem, unverkapseltem LHRH-Antagonisten wird entnommen. Nach der Zugabe von frischer Kochsalzlösung werden die Liposomen redispergiert und die Zentrifugation wird 5-mal wiederholt; abschließend werden die Liposomen auf 20 ml aufgefüllt. Nach einer Bestimmung des Wirkstoffgehalts mittels HPLC wird die Liposomendispersion mit Kochsalzlösung auf die Endkonzentration von 1,6 mg/ml HOE 013 verdünnt und in sterile Injektionsflaschen abgefüllt. Die volumenbezogeneTeilchengröße liegt im Mittel bei 2300 Nanometer, die Verkapselungseffizienz beträgt 20 %.

Beispiel 2

2 x 1 ml der Liposomen (entspr. einer Einmalgabe von 3200 μg HOE 013) aus Beispiel 1 werden subkutan weiblichen Ratten von ca. 200 g Körpergewicht injiziert. Als Kontrolle dient eine gleiche Testgruppe von Tieren, die nur Lösungsmittel (Kochsalzlösung) erhält (Placebo) und eine Gruppe von Tieren, die mit täglichen Gaben von LHRH-Antagonist-Lösung (60 μg) (in 5 %iger Mannitlösung) behandelt wird. Die Östrussuppression der Tiere wird durch wöchentlichen Östrusabstrich überprüft. Am Tag 35 wird die Konzentration von HOE 013 im Urin gemessen und die 24 h-Ausscheidung berechnet.

Die Ergebnisse (s. Tabelle 1) zeigen, daß die Tiere der Liposomengruppe im Gegensatz zu den beiden Kontrollgruppen nach 35 Tagen noch deutlich supprimiert sind. Die Exkretionsrate am Tag 35 (4,5 μg)

belegt eine signifikante Ausscheidung des Antagonisten im Falle der Liposomenpräparation. Diese Exkretionsrate ist mit der Plasmakonzentration eng korreliert.

Tabelle 1:     Zyklussuppression weiblicher Ratten nach subkutaner Injektion von LHRH-Antagonist HOE 013 bzw. Placebo

| Gruppe Nr. | Behandlung (Dosis) | Ratten mit Zyklussuppression/ Ratten pro Gruppe Tag der Vaginalcytologie | | | | | |
|---|---|---|---|---|---|---|---|
| | | 1 | 7 | 14 | 21 | 28 | 35 |
| 1 | Kontrolle (Placebo) | 0/11 | 0/11 | 0/11 | 0/11 | 0/11 | 0/11 |
| 2 | Kontrolle Tägliche Injektion (60 μg HOE 013 s.c.) | 0/11 | 0/11 | 0/11 | 0/11 | 0/11 | 0/11 |
| 3 | Liposomen (Einmalgabe von 3200 μg HOE 013 s.c.) | 0/8 | 8/8 | 8/8 | 8/8 | 8/8 | 8/8 |

Beispiel 3

40 mg LHRH-Antagonist HOE 013, 262 mg Dipalmitoyl-Phosphatidyl-Cholin (DPPC) (Phasenübergangstemperatur etwa 41° C) und 138 mg Cholesterol werden in 15 ml Methanol gelöst. Die Liposomenherstellung erfolgt analog Beispiel 1, das Volumen der wäßrigen Phase zur Filmablösung und Auffüllung der Liposomenpellets beträgt jedoch 4 ml.

Beispiel 4

40 mg LHRH-Antagonist HOE 013, 158,7 mg Dimyristoyl-Phosphatidyl-Cholin (DMPC) (Phasenübergangstemperatur etwa 23° C) und 41,3 mg Cholesterol werden wie in Beispiel 3 beschrieben zu Liposomen verarbeitet.

Beispiel 5

Die Liposomen aus den Beispielen 1, 3 und 4 werden bezüglich ihrer Freigabe in vitro getestet. Dazu werden 1 ml der Dispersion in Dialyseschläuche eingeschlossen, in einem Gefäß mit 10 ml Puffer (Tris-HCl 0,1 M, pH 7,4 isotonisiert mit NaCl) versetzt und bei 37° C unter Schütteln inkubiert. Die Pufferlösung wird täglich gewechselt und bezüglich des Gehaltes an HOE 013 analysiert. Die Ergebnisse (s. Tabelle 2) zeigen eine deutliche Abhängigkeit der Freigabe von der Zusammensetzung der Liposomenmembran.

Tabelle 2

| Tag | Peptidfreigabe in % | | |
| --- | --- | --- | --- |
| | Hydr. Eilezithin/CH (Beispiel 1) | DPPC/CH (Beispiel 3) | DMPC/CH (Beispiel 4) |
| 0 | 0 | 0 | 0 |
| 0,125 | 12,5 | 20,5 | 41,53 |
| 1 | 21,6 | 34,3 | 65,7 |
| 2 | 30,3 | 47,9 | 77,4 |
| 4 | 38,2 | 59,3 | 83,5 |
| 7 | 46,5 | 69,5 | 89,3 |
| 10 | 67,3 | 79,9 | 93,8 |
| 14 | 74,4 | 89,9 | 97,5 |
| 21 | 92,8 | 96,5 | 100,0 |
| 28 | 97,2 | 98,7 | |
| 35 | 98,1 | 99,8 | |

Beispiel 6

Statt HOE 013 werden 200 mg Buserelinacetat wie in Beispiel 1 beschrieben zu Liposomen verarbeitet. Die volumenbezogene Teilchengröße beträgt im Mittel 1800 nm, die Verkapselungseffizienz ist 10,6 %.

Beispiel 7

250 mg hydriertes Soyalezithin werden in 33,3 ml Diisopropylether und 16,7 ml Dichlormethan bei 40°C gelöst. 4 ml einer Lösung von 500 mg Hirudin (HBW 023) in 10 mM Phosphatpuffer pH 7,4 wird zugesetzt. Die Mischung wird 1 Minute im Ultraschallbad homogenisiert. Am Rotationsverdampfer wird das organische Lösungsmittel bei 55°C entfernt. Die gebildeten Liposomen werden 1 Stunde äquilibriert und dann durch 5 μm Membranfilter filtriert. Nach Entfernung des unverkapselten Anteils durch 3-malige Zentrifugation bei 8000 x g werden die Liposomenpellets auf 10 ml aufgefüllt. Die Verkapselungseffizienz beträgt 11,5 %.

Beispiel 8

135 mg hydriertes Eilezithin werden in 8 ml Diisopropylether und 4 ml Dichlormethan bei 40°C gelöst. 4 ml einer Lösung von 20 mg Ebiratid (HOE 427) in 10 mM Acetatpuffer pH 3,5 wird zugesetzt. Die Mischung wird 1 Minute im Ultraschallbad homogenisiert. Am Rotationsverdampfer wird das organische Lösungsmittel bei 55°C entfernt. Die gebildeten Liposomen werden 1 Stunde äquilibriert und dann durch 5 μm Membranfilter filtriert. Nach Entfernung des unverkapselten Anteils durch 3-malige Zentrifugation bei 16000 x g werden die Liposomenpellets auf 10 ml aufgefüllt. Die Verkapselungseffizienz beträgt 15 %.

Beispiel 9

Liposomen aus Beispiel 1 werden bei 4°C 12 Monate gelagert und dann bezüglich ihrer Lagerstabilität untersucht. Durch Zentrifugation bei 16000 UpM wird der nach Lagerung aus den Liposomen ins Dispersionsmittel (Wasser) freigesetzte Peptidanteil abgetrennt und mittels HPLC bestimmt. Nach 12 Monaten Lagerdauer sind 0,75 % der verkapselten Wirksubstanz freigesetzt, 99,25 % HOE 013 sind noch in den Liposomen gebunden. Die mittlere volumenbezogene Teilchengröße, bestimmt mit Photonenkorrelationsspektroskopie, ist mit 2300 nm gegenüber dem Ausgangswert unverändert.

Beispiel 10

2000 mg eines äquimolaren Gemisches von Dipalmitoyl-Phosphatidyl-Cholin (DPPC), hydriertem Eilezithin bzw. Eilezithin und Cholesterol (CH) werden in Methanol gelöst. Am Rotationsverdampfer wird das Lösungsmittel im Vakuum bei 55°C abgedampft. Die Lipidmatrix wird mit 20,0 ml einer Lösung von 200 mg HOE 013 in 5,4 %iger, wäßriger Mannitollösung bei 55°C abgelöst und über Nacht bei 50°C im Schüttelbad äquilibriert. Die gebildete Liposomendispersion wird durch ein 5 µm-Filter filtriert und dann auf etwa 20°C abgekühlt. Der unverkapselte Anteil wird durch Zentrifugation bei 1000 UpM über 10 min. abgetrennt. Die Zentrifugation wird nach Zusatz von 0,9 %iger Kochsalzlösung und Redispergierung 2-mal wiederholt.

Die gereinigte Liposomenfraktion wird auf die gewünschte HOE 013-Konzentration verdünnt und in sterile Injektionsflaschen abgefüllt. Die Verkapselungseffizienz beträgt

61,6 % für Liposomen aus DPPC/Cholesterol (50:50 Mol-%)
78,9 % für Liposomen aus hydrierten Eilezithin (HPC)/Cholesterol (50:50 Mol-%)
74,3 % für Liposomen aus Eilezithin (PC)/Cholesterol (50:50 Mol-%).

Beispiel 11

Liposomen aus Beispiel 10 werden weiblichen Ratten von 190-200 g mittleren Körpergewichts s.c. injiziert. Die Dosis beträgt 7,2 mg HOE 013 pro Tier. An festgelegten Zeitpunkten wird die Hemmung der Zyklen gegenüber einer Kontroll-Gruppe durch vaginale Zytologie ermittelt. Das Intervall der mittleren Östrus-Suppression beträgt

14 Tage für PC/CH-Liposomen (Gruppe 2)
34 Tage für HPC/CH-Liposomen (Gruppe 3)
48 Tage für DPPC/CH-Liposomen (Gruppe 4)

**Tabelle 3: Zyklussuppression weiblicher Ratten nach s.c. Injektion von 7,2 mg HOE 013 pro Tier**

**Gruppe**    **Ratten mit Zyklussuppression/Ratten pro Gruppe**

**Tag nach der Injektion**

| Gruppe | 0 | 1 | 2 | 3 | 6 | 7 | 8 | 9 | 12 | 14 | 16 | 21 | 23 | 28 | 31 | 34 | 37 | 41 | 44 | 48 |
|---|---|---|---|---|---|---|---|---|---|---|---|---|---|---|---|---|---|---|---|---|
| 1 (Kontrolle) | 0/8 | 0/8 | 0/8 | 0/8 | 0/8 | 0/8 | 0/8 | 0/8 | 0/8 | 0/8 | 0/8 | 0/8 | 0/8 | 0/8 | 0/8 | 0/8 | 0/8 | 0/8 | 0/8 | 0/8 |
| 2 | 0/8 | 0/8 | 0/8 | 1/8 | 8/8 | 6/8 | 8/8 | 8/8 | 8/8 | 8/8 | 8/8 | 7/8 | 7/8 | 7/8 | 6/8 | 6/8 | 3/8 | 2/8 | 2/8 | 0/8 |
| 3 | 0/7 | 0/7 | 0/7 | 1/7 | 6/7 | 5/7 | 7/7 | 6/7 | 7/7 | 7/7 | 7/7 | 7/7 | 6/7 | 5/7 | 5/7 | 5/7 | 4/7 | 5/7 | 3/7 | 3/7 |
| 4 | 1/8 | 1/8 | 2/8 | 5/8 | 7/8 | 8/8 | 7/8 | 6/8 | 7/8 | 4/8 | 3/8 | 2/8 | 1/8 | 2/8 | 3/8 | 4/8 | 0/8 | 0/8 | 0/8 | 0/8 |

Beispiel 12

3,37 g hydriertes Eilecithin und 1,63 g Cholesterol werden in 100 ml Methanol gelöst und 30 Minuten bei 60°C am Rotationsverdampfer zu einem Lipidfilm eingedampft. Nach dem Zusatz von Glasperlen werden 100 ml auf 60°C temperierte Mannitlösung (5,4 %) zugesetzt und der Film 60 Minuten unter Rotieren des Kolbens am Rotationsverdampfer bei 60°C abgelöst.

Die Liposomendispersion wird in einem Nanojet (Fa. Verstallen) 15 Minuten bei Spaltbreite 10 und einer Temperatur von 60° C behandelt. Die gebildeten kleinen Liposomen werden durch 0,2 um Membranfilter filtriert, nach dem Abkühlen in Injektionsfläschchen abgefüllt und anschließend lyophilisiert.

Zur Rekonstitution werden die Lyophilisate mit einer Lösung von 1 mg HOE 013 pro ml Aqua ad Injektabilia versetzt und bei 60° C geschüttelt.

Die Abtrennhung des unverkapselten Anteils erfolgt entsprechend Beispiel 1 durch wiederholte Zentrifugation. Der liposomengebundene Anteil beträgt 28,9 % des Wirkstoffs.

## Patentansprüche

1. Liposomenpräparate für Peptide mit verlängerter Peptidfreisetzung, dadurch gekennzeichnet, daß die Peptide ein Molekulargewicht zwischen etwa 500 und 10000 aufweisen, die Phospholipidkomponente der Liposomenmembran eine Phasenübergangstemperatur von mindestens 20° C besitzt und überwiegend gesättigte Fettsäuren enthält und daß die Wirksamkeit nach s.c. oder i.m. Injektion mehr als 14 Tage anhält.

2. Liposomenpräparate gemäß Anspruch 1, dadurch gekennzeichnet, daß eine oder mehrere der folgenden Bedingungen erfüllt sind
   a) das Peptid ein LHRH-Analogon, ein Bradykinin-Antagonist, HOE 427 oder ein Hirudin-Derivat ist,
   b) die Phasenübergangstemperatur der Phospholipidkomponente über 30° C liegt,
   c) die überwiegend gesättigten Fettsäuren der Phopholipidkomponente eine Kettenlänge von mindestens 14 Kohlenstoffatomen aufweisen,
   d) die Liposomenmembran einen Steroidzusatz enthält,
   e) die Liposomen eine mittlere volumenbezogene Teilchengröße von mindestens 600 bis 10000 Nanometer besitzen,
   f) die Wirksamkeit mindestens 20 Tage anhält.

3. Liposomenpräparate gemäß Anspruch 1, dadurch gekennzeichnet, daß eine oder mehrere der folgenden Bedingungen erfüllt sind
   a) das Peptid eine LHRH-Analogon, HOE 140, HOE 427 oder HBW 023 ist,
   b) die Phasenübergangstemperatur der Phospholipidkomponente mindestens 37° C beträgt,
   c) die überwiegend gesättigten Fettsäuren der Phospholipidkomponente eine Kettenlänge von mindestens 14 Kohlenstoffatomen aufweisen,
   d) die Liposomenmembran einen Steroidzusatz enthält,
   e) die Liposomen eine mittlere volumenbezogene Teilchengröße von mindestens 600 bis 10000 Nanometer besitzen,
   f) die Wirksamkeit mindestens 30 Tage anhält.

4. Liposomenpräparat gemäß Anspruch 1, dadurch gekennzeichnet, daß eine oder mehrere der folgenden Bedingungen erfüllt sind
   a) das Peptid Buserelinacetat oder HOE 013 ist,
   b) die Phasenübergangstemperatur mindestens 37° C beträgt,
   c) die Phospholipidkomponente Dipalmitoyl-Phosphatidyl-Cholin (DPPC) oder hydriertes Lecithin aus natürlichen Quellen ist,
   d) die Membran einen Cholesterolzusatz enthält,
   e) die Liposomen eine mittlere volumenbezogene Teilchengröße von mindestens 600 bis 10000 Nanometer besitzen,
   f) die Wirksamkeit mindestens 30 Tage anhält.

5. Liposomenpräparate gemäß Anspruch 1, dadurch gekennzeichnet, daß zusätzliche Ladungsträger im Membranmaterial enthalten sind.

6. Liposomenpräparate gemäß Anspruch 1, dadurch gekennzeichnet, daß sie Antioxidantien und andere Hilfsstoffe mit stabilisierenden oder freigabebeeinflussenden Eigenschaften enthalten.

7. Verwendung von Liposomenpräparaten gemäß Anspruch 1, zu s.c. oder i.m. Injektion.

8. Verfahren zur Herstellung von Liposomenpräparaten gemäß Anspruch 1, dadurch gekennzeichnet, daß man

a)

α) die Phospholipidkomponente, und gegebenenfalls lipophile Zusätze, in einem geeigneten organischen Lösungsmittel löst, das Lösungsmittel entfernt und die erhaltene Lipidmatrix nach Zusatz einer wäßrigen Lösung des Peptids zur Liposomenbildung ablöst, wobei die Ablösung oberhalb der Phasenübergangstemperatur der Phospholipidkomponente erfolgt oder

β) die Phospholipidkomponente, und gegebenenfalls lipophile Zusätze, sowie das Peptid in einem geeigneten organischen Lösungsmittel löst, das Lösungsmittel entfernt und die erhaltene Lipidmatrix mit einem wäßrigen Medium ablöst, wobei die Ablösung oberhalb der Phasenübergangstemperatur der Phospholipidkomponente erfolgt, oder

γ) die Phospholipidkomponente, und gegebenenfalls lipophile Zusätze, in einem flüchtigen organischen Lösungsmittel löst und eine wäßrige, mit der organischen Phase nicht mischbare Peptidlösung zusetzt, das erhaltene Zwei-Phasensystem durch Homogenisierung oberhalb der Phasenübergangstemperatur der Phospholipidkomponente in eine stabile Emulsion überführt und das organische Lösungsmittel unter Bildung von Liposomen entfernt

und die nach den Methoden α bis γ erhaltenen Liposomendispersionen, gegebenenfalls nach Homogenisierung und Äquilibrierung, auf den gewünschten Peptidgehalt einstellt, abfüllt, und gegebenenfalls gefriertrocknet, oder

b) ein Lyophilisat von Peptid-freien Liposomen nach Methoden α, β oder γ herstellt und eine wäßrige Peptidlösung in einen geeigneten Gefäß abfüllt, wobei Lyophilisat und Peptidlösung vor Applikation vereinigt werden.